(19) Europäisches Patentamt
European Patent Office
Office européen des brevets

(11) **EP 1 558 187 B1**

(12) **EUROPEAN PATENT SPECIFICATION**

(45) Date of publication and mention
of the grant of the patent:
**11.04.2007 Bulletin 2007/15**

(21) Application number: **03781720.2**

(22) Date of filing: **03.11.2003**

(51) Int Cl.:
**A61F 7/02** (2006.01)

(86) International application number:
**PCT/US2003/034940**

(87) International publication number:
**WO 2004/043312 (27.05.2004 Gazette 2004/22)**

(54) **KITS COMPRISING BODY COMPRESS AND RELEASABLY ATTACHABLE THERMIC DEVICE**

KITS BESTEHEND AUS KÖRPERKOMPRESSE UND LÖSBAR BEFESTIGBARE THERMISCHE VORRICHTUNG

KITS COMPRENANT UNE COMPRESSE CORPORELLE ET UN DISPOSITIF THERMIQUE COLLANT DECOLLABLE

(84) Designated Contracting States:
**AT BE BG CH CY CZ DE DK EE ES FI FR GB GR HU IE IT LI LU MC NL PT RO SE SI SK TR**

(30) Priority: **06.11.2002 US 424192 P**

(43) Date of publication of application:
**03.08.2005 Bulletin 2005/31**

(73) Proprietor: **THE PROCTER & GAMBLE COMPANY**
**Cincinnati, Ohio 45202 (US)**

(72) Inventors:
• **CIPRA, Andrew, Raymond**
**Cincinnati, OH 45249 (US)**
• **PACE, Gwen, Elaine**
**Morrow, OH 45152 (US)**

(74) Representative: **Clemo, Nicholas Graham et al**
**Procter & Gamble Technical Centres Limited**
**Patent Department**
**Rusham Park,**
**Whitehall Lane**
**Egham, Surrey TW20 9NW (GB)**

(56) References cited:
WO-A-84/02071          WO-A-97/49361
DE-U- 9 207 954        US-A- 5 020 711
US-A- 5 984 995

**Description**

FIELD OF THE INVENTION

The present invention relates to kits comprising one or more body compresses and one or more thermic devices, wherein the kit comprises means for releasably attaching the thermic device to one of the body compresses. The thermic devices are expendible, and may be disposed of subsequent to use, while the body compresses are more durable and capable of reuse.

BACKGROUND OF THE INVENTION

A common method of treating temporary or chronic pain is by application of heat to the afflicted area. Such heat treatments are used as a means of therapy for conditions which include aches, Stiffness in muscles and joints, nerve pain, rheumatism, and the like. Typically, the method for relieving pain using beat treatments has been topical application of relatively high heat, *e.g.*, greater than about 40 °C for a short period of time, *e.g.*, from about twenty minutes to about one hour.

While elastic compression bandages have been used to help stabilize afflicted areas during injury healing, heating pads, whirlpools, hot towels, hot water bottles, hot packs, and the like have been commonly used to apply heat to the area to relieve the pain of joint injury. However, many of these devices are inconvenient for use on a regular and extended basis because the heat energy may not be immediately available when needed or released in a controllable manner. That is, many of these thermal units or devices do not provide long lasting heat and also do not maintain a consistent temperature over long periods of time. Proper positioning of the thermal energy also may not be maintainable during joint flexure. In general, the beneficial therapeutic effects from this administration of heat diminish after the heat source is removed.

Disposable heat packs based on iron oxidation, such as those described in U.S. Patent Nos. 4,366,804; 4,649,895; 5,046,479 and Re. 32,026, are known. However, such devices have not been proven satisfactory because many of these devices are bulky, cannot maintain a consistent and controlled temperature, present difficulty staying in place during use, and/or have unsatisfactory physical dimensions that hinder their efficacy. Specifically, such devices cannot be easily incorporated into wraps that comfortably and reliably conform to various body contours, and hence, deliver inconsistent, inconvenient and/or uncomfortable heat application to the body.

US 5,020,711 discloses a pouch for reusable hot/cold packs for treatment of muscle injuries. DE 0920795401 discloses an orthopedic support bandage that may have heating devices attached. WO 84/02071A discloses a compression bandage having a releasably attached bag heating element.

Very recently, improved disposable body wraps have been described in such references as U.S. Patent Nos. 5,728,057; 5,728,058; 5,860,945; 6,048,326; 5,728,146; 5,735,889; 6,102,937; 6,123,717; 5,925,072; 6,074,413; 5,741,318; 5,980,562; 5,674,270; 5,837,005; 6,096,067; 6,019,782; 5,906,637; 6,024,761; 5,904,710; and 6,336,935; WO 98/29064; WO 97/01312; WO 97/01310; WO 97/49361; WO 98/29063; WO 99/09917; WO 99/09918; and WO 01/19302. These references disclose disposable body wraps comprising a plurality of heat cells, wherein the body wraps deliver consistent, long-lasting thermal treatment to the afflicted area of the body. The wraps described in these referenced, and the technology incorporated therein, have provided important advances in the art.

It would be, however, desirable to provide wraps that are not immediately disposable upon expenditure of the heat or other thermic source. Recently, wraps such as HEAT ZONE®, commercially available from AccuFitness, Englewood, CO, have been provided that contain a semi-durable body wrap having a pocket for the placement of a single "heat pad" containing an exothermic composition. While these products diminish the disposable nature of the overall product, the heat pads are subject to movement and displacement when contained in the pocket. This results in decreased efficacy and utility of the overall wrap, caused by failure of the product to consistently deliver efficacious heat therapy to the area in need of treatment.

The presently inventive kits provide advantages relative to those provided in the art. Indeed, the present kits provide body compresses which consistently deliver the thermic source to the area in need of treatment, as the thermic source is releasably affixed to the compress during use. These and other advantages of the knee wraps are described more particularly herein.

SUMMARY OF THE INVENTION

The present invention is directed to kits comprising a body compress and a releasably attachable thermic device according to claim 5.

Exemplary body compresses include knee wraps, neck wraps, back wraps, and menstrual pain relief compresses. The releasably attachable thermic devices are preferably disposable. Therefore, continued use of the preferred durable body compress is possible, with interchange of various disposable thermic devices upon expenditure of each thermic device.

The present invention may be used to treat pain selected from acute muscular, acute skeletal, acute referred, recurrent muscular, recurrent skeletal, recurrent referred, chronic muscular, chronic skeletal, chronic referred pain, and combinations thereof, by applying the foregoing body compress, having one or more thermic devices releasably attached thereto, to the area in need of treatment or, alternatively, applying the foregoing body compress to the area in need of treatment followed by releasable attachment of one or more thermic devices to the applied body compress.

BRIEF DESCRIPTION OF THE DRAWINGS

While the specification concludes with claims that particularly point out and distinctly claim the present invention, it is believed that the present invention is further understood from the following description of preferred embodiments, taken in conjunction with the accompanying drawings, wherein:

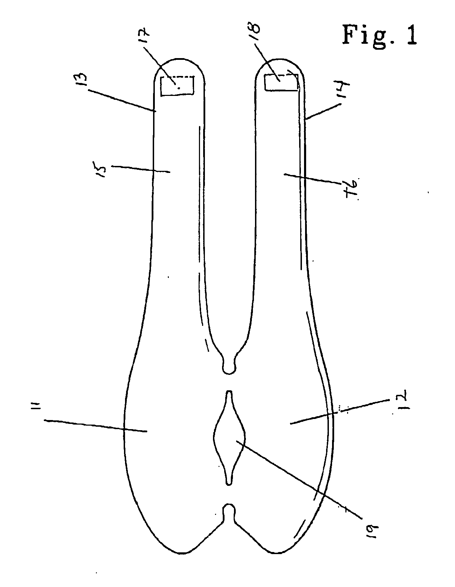

FIG. 1 is a top view of a preferred embodiment of a body compress utilized in the present kits, wherein the body compress is a knee wrap.

DETAILED DESCRIPTION OF THE INVENTION

Various documents including, for example, publications and patents, are recited throughout this disclosure.

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (*e.g.*, those obtained from a different source under a different name or reference number) to those referenced by trade name may be substituted and utilized in the descriptions herein.

As used herein, the term "mammal" means vertebrate mammals. Preferred mammals are humans and companion animals (*e.g.*, domestic cats, dogs, horses, cows, or other similar animals). The most preferred mammals are humans.

As used herein, the term "plurality" with reference to a given noun means more than one, preferably more than two, more preferably more than three, and most preferably more than four units of the given noun.

Kits and Methods of the Present Invention

The present kits include those comprising:

(a) one or more body compresses; and
(b) one or more thermic devices each comprising a plurality of exothermic or endothermic thermic cells; and
(c) means for releasably attaching one or more of the thermic devices to one of the body compresses, comprising a pressure sensitive adhesive.

The kits may comprise any number of body compresses and thermic devices, as desired. Since the body compresses are intended as durable or semi-durable devices, typical kits may comprise, for example, one body compress and one, or a plurality, of thermic devices. This is convenient for the user, who may choose to use the body compress on multiple occasions, but dispose of a given thermic device subsequent to expenditure of the device. Typically, as described below, each thermic device is separately contained in a secondary package, such that the body compress and one or a plurality of thermic devices are each separately contained in secondary packages and then finally packaged as a single kit. Packaging for the single kit may be any of a variety of types, for example, a carton or box containing the members of the kit.

Each of the elements of this invention, as well as preferred, optional embodiments, is described further herein:

The Body Compress

The kits in accordance with this invention comprise one or more body compresses. The body compress provides means for compressing or wrapping the afflicted area of the body, wherein this afflicted area is in need of application of heating or cooling for therapeutic or other purposes.

The body compress may be any of a variety of wraps, pads, compresses, or the like which may be applied to the mammalian body. Indeed, as an advantage of the present invention, the body compresses herein may be utilized on an ongoing basis since the thermic devices, releasably attachable to the body compress, typically contain components which render the thermic device expendable (as further described below with reference to the thermic devices). Accordingly, the body compress may be reused subsequent to expenditure of the thermic device, by releasing the thermic device from the body compress as desired. The body compress is preferably such that it is stored for later use and/or hand or machine washable to maintain the integrity of the compress.

For example, the body compress may be an ACE® bandage or wrap (commercially available from Becton Dickenson of Franklin Lakes, NJ), or another like bandage or wrap. Alternatively or additionally, the body compress comprises any stretch material including, but not limited to, natural and synthetic rubbers, styrene block copolymers, polyurethane, metallocene-catalyzed polyethylene, and the like. These materials may optionally include foams, nonwoven materials, knits, films, elastomeric scrims, films, strands, ribbons, tapes, structural elastic-like films, laminates of nonwovens and the aforementioned materials, and the like.

In another embodiment of the present invention, the body compress may have the design, shape, components, or other features of known thermal wraps or pads, such as set forth in U.S. Patent Nos. 5,728,057; 5,728,058; 5,860,945; 6,048,326; 5,728,146; 5,735,889; 6,102,937; 6,123,717; 5,925,072; 6,074,413; 5,741,318;

5,980,562; 5,674,270; 5,837,005; 6,096,067; 6,019,782; 5,906,637; 6,024,761; 5,904,710; or 6,336,935; WO 98/29064; WO 97/01312; WO 97/01310; WO 97/49361; WO 98/29063; WO 99/09917; WO 99/09918; or WO 01/19302; except that the body compresses utilized herein are most preferably free of the thermic packs and heat cells described in these foregoing references in order to impart durability to the compress. Thus, the body compresses may comprise a variety of various layers of material, and designed in specific shapes and orientations such thai it may be used on the part of the body in need of treatment. For example, these references collectively describe knee wraps, neck wraps, back wraps, menstrual pain relief compresses, and the like.

Since the embodiments herein comprise thermic devices comprising one or more exothermic or endothermic compositions, it is preferred that the body compresses herein are permeable to air. That is, the body compress is preferably permeable to air in order that the compress does not inhibit any exothermic or endothermic reaction within the thermic device when the thermic device is releasably attached to the compress.

In order to impart added structure and further durability to the body compress, one or more optional stays may be included. Thus, the stays may serve as resilient stiffeners. For example, such stays may be embedded internally in the layers of the material of the body compress. Alternatively, the stays may be positioned on an outer surface of the body compress.

Exemplary stays include stripes of glue which are positioned to permit the body compress to bend and conform with body movement during use, yet minimizes bunching and deformation. Exemplary glue stays include HL1460-X, commercially available from Fuller or Minneapolis, MN. Beads of about 5 mm in diameter may be extruded on, or within, the body compress with a conventional hot melt glue gun and then calendered or flattened via a compression roll to the desired thickness. Thickness will typically determine stiffness of the stays. Preferably, the thickness of the stays is from about 0.3 mm to about 5 mm. Wherein the stays are applied within the body compress, other layers of the body compress are then added to provide the finished compress. Preferred materials for the stays include, for example, polyethylene or polypropylene. Typically the stays, independently, have a thickness of from about 0.5 mm to about 2 mm, most preferably from about 0.75 mm to about 1.25 mm. The stays may be hot-melt extruded or printed directly onto the body compress or cut from sheeting and individually affixed to the compress.

Alternatively, stays may be made of rigid plastic or metal. With rigid plastic and metal stays, pockets may optionally be sewn into the compress, and then individual stays are formed and installed.

The body compress is designed in the desired form, depending upon needed treatment. This is illustrated herein by a knee wrap, set forth herein as Fig. 1. Referring now to Fig. 1, the body compress (in the form of a knee wrap) comprises a first attachment zone 11 and a second attachment zone 12, wherein each of the attachment zones are suitable for releasable attachment of a thermic device. The body compress further comprises upper strap portion 13 and lower strap portion 14, which are suitable for encircling the user's knee. Strap portions 13 and 14 comprise first elastic member 15 and second elastic member 16, respectively, which allow appropriate fit depending upon the needs of the particular user. Strap portions 13 and 14 further comprise first hook members 17 and second hook members 18 to allow the body compress to be secured around the user's knee during use. Aperture 19 is suitable for allowing protrusion of the user's patella.

The ordinarily skilled artisan will understand that Fig. 1, illustrating a body compress in the form of a knee wrap, is exemplary only and that other forms of wraps may be utilized. For example, the body compress used herein may be a neck wrap, back wrap, menstrual pain relief compress, or the like. Guidance for various compress forms may be found in U.S. Patent Nos. 5,728,057; 5,728,058; 5,860,945; 6,048,326; 5,728,146; 5,735,889; 6,102,937; 6,123,717; 5,925,072; 6,074,413; 5,741,318; 5,980,562; 5,674,270; 5,837,005; 6,096,067; 6,019,782; 5,906,637; 6,024,761; 5,904,710; or 6.336.935; WO 98/29064; WO 97/01312; WO 97/01310; WO 97/49361; WO 98/29063; WO 99/09917; WO 99/09918; or WO 01/19302; however, it is again noted that the body compresses described herein are most preferably free of the thermic packs and heat cells described in these foregoing references in order to impart durability to the compress.

The Thermic Device

The thermic device utilized herein is suitable for providing heat or cold, as desired or needed, to the user's joint when releasably attached to the body compress. As such, the thermic device preferably comprises one or more, preferably a plurality, of thermic cells.

The thermic devices comprise one or more thermic cells comprising an exothermic or endothermic composition, as applicable. Thermic devices comprising a plurality of thermic cells are preferred herein, particularly wherein such cells are spaced apart and fixed within or to the structure of the thermic device. The cells are a unified structure, comprising the exothermic or endothermic composition, enclosed within two layers, wherein at least one layer may be oxygen permeable, capable of providing long lasting heating or cooling with improved temperature control, and having specific physical dimensions and fill characteristics. These cells can be used as individual temperature control units, or in a thermal pack comprising a plurality of individual cells which can also be easily incorporated into disposable body wraps, pads, and the like.

In a particularly preferred embodiment of the present invention, the thermic devices are readily adaptable for use herein as described in, for example, U.S. Patent Nos. 6,020,040 and 6,146,732; and WO 98/29066. In this embodiment, the thermic devices typically comprise at least one continuous layer of a material, which preferably exhibits specific thermophysical properties and a plurality of individual heat cells, which preferably comprise an exothermic composition, spaced apart and fixed within or to the structure of the disposable thermic device.

In describing these preferred thermic devices, it is useful to define a number of terms used herein:

"Agglomerated pre-compaction composition" means the mixture of dry powdered ingredients, comprising iron powder, carbonaceous powder, metal salt(s), water-holding agent(s), agglomeration aid(s), and dry binder(s) prior to direct compaction. "Direct compaction" means a dry powder mixture is blended, compressed, and formed into pellets, tablets, or slugs without the use of typical wet binders/ solutions to adhere the particulate(s) together. Alternatively, the dry powder mixture is blended and roll compacted or slugged, followed by milling and screening, creating directly compacted granules. Direct compaction may also be known as dry compaction.

"Heating elements" means the exothermic, direct compacted, dry agglomerated pre-compaction composition formed into compaction articles, such as granules, pellets, slugs, and/or tablets capable of generating heat, after an aqueous solution such as water or brine (salt solution) is added, by the exothermic oxidation reaction of iron. Agglomeration granules of said agglomerated pre-compaction composition are also included as heating elements herein.

"Fill volume" means the volume of the particulate composition or the compacted, water-swelled, heating element in the filled heat cell. "Void volume" means the volume of the cell left unfilled by the particulate composition or the compacted, water-swelled, heating element in a finished heat cell, not including the unfilled space within a tablet comprising a hole or reservoir, in a finished heat cell, measured without differential pressure in the heat cell and without additional stretching or deformation of the substrate material. "Cell volume" means the fill volume plus the void volume of the heat cell.

"Continuous layer or layers" means one or more layers of a material which may be uninterrupted or partially, but not completely, interrupted by another material, holes, perforations, and the like, across its length and/or width.

"Rigid" means the property of a material wherein the material may be flexible, yet is substantially stiff and unyielding, and which does not form fold lines in response to gravitational pull or other modest forces.

"Semirigid material" means a material which is rigid to some degree or in some parts, i.e., having at least two-dimensional drape at a temperature of about 25°C, and exhibits a toughness to maintain structural support of the heat cells in an unsupported format, and/or to prevent unacceptable stretching of structures of the material during processing or use and/or to deter easy access to heat cell contents while still maintaining good overall drape characteristics when heated.

"Two-dimensional drape" means drape which occurs across a continuous layer or layers, across a thermic device, or across a select region of a layer or layers, or thermic device, exclusively along one axis, *i.e.*, one fold line forms, at the expense of other fold lines in response to gravitational pull or other modest forces.

"Three dimensional drape" means drape which simultaneously occurs across a continuous layer or layers, across a thermic device, or across a select region of a layer or layers, or thermic device, along two or more axes, *i.e.*, two or more fold lines form, in response to gravitational pull or other modest forces.

"Fold lines" means the line along which a material forms a temporary or permanent crease, ridge, or crest in response to gravitational pull or other modest forces.

The thermic devices of this embodiment comprise at least one continuous layer of a material, which preferably exhibits specific thermophysical properties. The material of the at least one continuous layer is preferably semirigid when at room temperature, for example 25 °C or below, but softens and becomes substantially less rigid when heated to 35 °C or greater. Therefore, when heat cells, which are fixed within or to the unified structure of the thermic device, are active, that is at a heat cell temperature of from 35°C to 60 °C, preferably from 35 °C to 50 °C, more preferably from 35 °C to 45 °C, and most preferably from 35 °C to 40 °C, the narrow portion of the continuous layer or layers of material immediately surrounding each heat cell preferably softens and acts as a hinge between the heat cells and remaining more rigid portions of the continuous layer or layers, bending preferentially more than either the heat cells or cooler, more rigid portions. This results in a thermic device which possesses sufficient rigidity to maintain structural support of the heat cells and prevents undesirable stretching of structures of the continuous layer or layers during processing or use, while still maintaining good overall drape characteristics when heated. The thermic devices easily adapt to a wide variety of body compress designs, provides consistent, convenient, and comfortable heat application, and an excellent conformity with body forms, while retaining sufficient rigidity to deter easy access to the heat cell contents.

The continuous layer or layers preferably comprises a material which is semirigid at a temperature of 25 °C and which softens, *i.e.,* becomes substantially less rigid, at a temperature of 35 °C or greater. That is, the material has a tensile strength, within the elastic deformation range of the material, of 0.7 g/mm$^2$ or greater, preferably 0.85 g/mm$^2$ or greater, more preferably about 1 g/mm$^2$ or greater, at 25 °C and a tensile strength substantially less at 35 °C or greater. "Substantially less," as used in this context, means that the tensile strength of the material at 35 °C, or greater, is statistically significantly less than the tensile strength at 25 °C, at an appropriate statistical confidence (*i.e.,* 95%) and power (*i.e,* ≥ 90%).

Typically, the tensile strength is measured using a simple tensile test on an electronic tensile test apparatus, such as a universal constant rate elongation tensile testing machine with computer, Instron Engineering Corp., Canton, MA. Any standard tensile test may be used, for example, material samples are cut into strips having a width of 2.54 cm (about 1 inch) and a length of from 7.5 cm to 10 cm (about 3 to about 4 inches). The ends of the strips are placed into the jaws of the apparatus with enough tension to eliminate any slack, but without loading the load cell. The temperature of the sample is then allowed to stabilize at the desired test temperature. The load cell of the apparatus is set for 22.7 kg (50 pound) load, the elongation set for 5 nun, and the crosshead speed is set for 50 cm/min. The apparatus is started and the tensile strength data is collected by the computer. The sample is then removed from the apparatus.

The tensile strength may be calculated as the slope of the tensile load vs. the extension during clastic deformation of the materials using the equation:

$$m = (L/E)$$

Where m = the slope in g/mm$^2$ during elastic deformation;
L = the load at extension in g/mm; and
E = the extension in mm.

The continuous layer or layers also preferably comprises at least two-dimensional drape at 25 °C, *i.e.,* a single fold or crease occurs in the material along a single axis, and preferably three-dimensional drape at 35 °C or greater, *i.e.,* two or more folds or creases occur along multiple axes. Drape may be determined by placing and centering a square sample, for example 30 cm by 30 cm (12 inches by 12 inches), of material on the end of a cylindrical shaft with a pointed end, allowing the material to drape due to gravitational forces, and the number of fold lines counted. Materials that exhibit one-dimensional drape, *i.e.,* have no folds or creases in any direction, are determined to be rigid, while materials that exhibit at least two-dimensional drape, *i.e.,* have at least one fold or crease line forming along at least one axis are determined to be semirigid.

Different materials may be capable of satisfying the specified requirements described above provided that the thickness is adjusted accordingly. Such materials may include, but are not limited to, polyethylene, polypropylene, nylon, polyester, polyvinyl chloride, polyvinylidene chloride, polyurethane, polystyrene, saponified ethylene-vinyl acetate copolymer, ethylene-vinyl acetate copolymer, natural rubber, reclaimed rubber, synthetic rubber, and mixtures thereof. These materials may be used alone, preferably extruded, more preferably coextruded, most preferably coextruded with a low melt temperature polymer including, but not limited to, ethylene vinyl acetate copolymer, low density polyethylene, and mixtures thereof.

The continuous layer or layers of material preferably comprises polypropylene, more preferably a coextruded material comprising polypropylene, most preferably a coextruded material wherein a first side comprises polypropylene, preferably from 10% to 90%, more preferably from 40% to 60%, of the total thickness of the material, and a second side comprises a tie-layer of a low melt temperature copolymer, preferably EVA. The continuous layer or layers of material preferably comprise a combined basis weight thickness of less than 50 μm, more preferably less than 40 μm most preferably less than 30 μm.

A particularly suitable and preferred material for the continuous layer or layers is a coextruded material having a first side of polypropylene and a second side of EVA having a total material thickness of from 20 μm to 30 μm, preferably 25 μm (1 mL), wherein the polypropylene comprises 50% and the EVA tie-layer comprises 50% of the total material thickness. This material is available from Clopay Plastic Products, Cincinnati, Ohio, as P18-3161. When the polypropylene/EVA coextruded material is used to make the thermic device and/or heat cells, the polypropylene side is oriented to the outside (*i.e.,* away from the exothermic composition).

Good overall drape characteristics and/or excellent conformity with various body forms, and/or increased structural support to the thermic device, may also be achieved by selectively placing the heat cells into positions fixed within or to the unified structure of the thermic devices relative to each other which are sufficiently close so as to block some or all possible axes across the material of the continuous layer or layers which otherwise would have passed uninterrupted between the heat cells, through the thermic devices, or select regions thereof, to minimize or eliminate undesirable, uninterrupted fold lines. That is, placement of the heat cells into positions relative to each other which are sufficiently close so that the number of axes which pass uninterrupted, between the heat cells, is selectively controlled, such that the continuous layer or layers of the disposable thermic devices, or select regions thereof, preferably folds along a multiplicity of short interconnected fold lines oriented in a number of different directions relative to each other. Folding along a multiplicity of interconnected fold lines results in thermic devices which have good overall drape characteristics, readily conform with various body forms, and/or have increased structural support of the heat cell matrix.

Because the heat cells are not readily flexible, the spacing between the heat cells provides the preferred benefits and may be determined, when selectively placing the heat cells within or fixed to the unified structure of the thermic devices of the present invention, wherein at least one heat cell of four adjacent heat cells, whose centers form a quadrilateral pattern, blocks one or more axes that could otherwise form at least one fold line tangential to the edges of one or more pairings of the remaining three heat cells in the quadrilateral pattern. Preferably, the spacing between at least one heat cell of the four adjacent heat cells and each of the beat cells of the one or more pairings of the remaining heat cells in the quadrilateral pattern may be calculated using the equation:

$$s \leq (W_q/2) * 0.75$$

Where s = the closest distance between the heat cells; and

$W_q$ = the measurement of the smallest diameter of the smallest diameter heat cell within the quadrilateral pattern.

Alternatively, the spacing between the heat cells may be determined wherein, at least one heat cell of three adjacent heat cells, whose centers form a triangular pattern, blocks one or more axes that could otherwise form at least one fold line tangential to the edges of the remaining pair of heat cells in the triangular pattern formed by the three heat cells. Most preferably, the spacing between the at least one heat cell of the three adjacent heat cells and each heat cell of the remaining pair of heat cells in the triangular pattern may be calculated using the equation:

$$s \leq (W_t/2) * 0.3$$

Where s = the closest distance between the heat cells; and

$W_t$ = the measurement of the smallest diameter of the smallest diameter heat cell within the triangular pattern.

Different materials may be capable of satisfying the above specified requirements. Such materials may include, but are not limited to, those materials mentioned above.

A most preferred embodiment of the disposable thermic devices comprises at least one continuous layer of semirigid material having the thermophysical properties described above, and the heat cells fixed within or to the unified structure of the thermic device in positions relative to each other which are sufficiently close so as to block some or all possible axes across the material of the continuous layer(s), which otherwise would have passed uninterrupted between the heat cells, through the thermic devices, or select regions thereof, to minimize or eliminate undesirable, uninterrupted fold lines, as described above.

In this embodiment, the thermic devices comprise a plurality of individual heat cells fixed within or to the unified structure of the thermic device. These heat cells are spaced apart from each other and each heat cell functions independently of the rest of the heat cells. While the heat cells may comprise any suitable composition providing heat, such as exothermic compositions, microwaveable compositions, heat of crystallization compositions, and the like, the preferred heat cell contains a densely packed, particulate exothermic composition which substantially fills the available cell volume within the cell reducing any excess void volume thereby minimizing the ability of the particulate matter to shift within the cell. Alternatively, the exothermic composition may be compressed into a hard tablet or slug before being placed into each cell.

The preferred exothermic composition comprises a mix of chemical compounds that undergo an oxidation reaction during use. The mix of compounds typically comprises iron powder, carbon, a metal salt(s), and water. Mixtures of this type react when exposed to oxygen, providing heat for several hours. Preferably, the exothermic composition comprises a particulate mix of chemical compounds that undergo an oxidation reaction during use. Alternatively, the exothermic composition may also be formed into agglomerated granules, direct compacted into compaction articles such as granules, pellets, tablets, and/or slugs, and mixtures thereof. The mix of compounds typically comprises iron powder, carbon, a metal salt(s), and water. Mixtures of this type, which react when exposed to oxygen, provide heat for several hours.

Suitable sources for iron powder include cast iron powder, reduced iron powder, electrolytic iron powder, scrap iron powder, pig iron, wrought iron, various steels, iron alloys, and the like and treated varieties of these iron powders. There is no particular limitation to their purity, kind, and other properties, so long as it can be used to produce heat-generation with electrically conducting water and air. Typically, the exothermic composition comprises from 30% to 80% iron powder, more preferably from 50% to 70% iron powder, all by weight of the exothermic composition.

Carbonaceous material selected from the group consisting of activated carbon, non-activated carbon, and mixtures thereof may be used in the exothermic compositions. Active carbon prepared from coconut shell, wood, charcoal, coal, bone coal, and the like are useful, but those prepared from other raw materials such as animal products, natural gas, fats, oils and resins are also useful in the particulate exothermic composition optionally used herein. There is no limitation to the kinds of active carbon used, however, the preferred active carbon has superior water holding capabilities and the different carbons way be blended to reduce cost. Therefore, mixtures of the above carbons are useful in the present invention as well. Typically, the composition comprises from 3% to 25% carbonaceous material, more preferably from 8% to 20% carbonaceous material, and most preferably from 9% to 15% carbonaceous material, all by weight of the composition.

The metal salts useful in the particular exothermic composition include sulfates such as ferric sulfate, potassium sulfate, sodium sulfate, manganese sulfate, magnesium sulfate; and chlorides such as cupric chloride, potassium chloride, sodium chloride, calcium chloride, manganese chloride, magnesium chloride and cuprous chloride. Also, carbonate salts, acetate salts, nitrates, nitrites and other salts can be used. In general, several suitable alkali, alkaline earth, and transition metal salts exist which can also be used, alone or in combination, to sustain the corrosive reaction of iron. The preferred metal salts are sodium chloride, cupric chloride, and mixtures thereof. Typically, the exothermic composition comprises from 0.5% to 10%, more preferably from 1.0% to 5% by weight, metal salts, all by weight of the exothermic composition.

The water used in the particulate exothermic composition may be from any appropriate source. There is no particular limitation to its purity, kind, and the like. Typically, the exothermic composition comprises from 1% to 40%, more preferably from 10% to 30%, water, all by weight of the exothermic composition.

Additional water-holding materials may also be added as appropriate. Useful additional water-holding materials include vermiculite, porous silicates, wood powder, wood flour, cotton cloth having a large amount of fluffs, short fibers of cotton, paper scrap, vegetable matter, super absorbent water-swellable or water-soluble polymers and resins, carboxymethylcellulose salts, and other porous materials having a large capillary function and hydrophilic property can be used. Typically, the exothermic composition comprises from 0.1% to 30%, more preferably from 0.5% to 20% by weight, and most preferably from 1% to 10%, water-holding materials, all by weight of the exothermic composition.

Other additional components include agglomeration aids such as gelatin, natural gums, cellulose derivatives, cellulose ethers and their derivatives, starch, modified starches, polyvinyl alcohols, polyvinylpyrrolidone, sodium alginates, polyols, glycols, corn syrup, sucrose syrup, sorbitol syrup and other polysaccharides and their derivatives, polyacrylamides, polyvinyloxoazolidone, and maltitol syrup; dry binders such as maltodextrin, sprayed lactose, co-crystallized sucrose and dextrin, modified dextrose, sorbitol, mannitol, microcrystalline cellulose, microfine cellulose, pre-gelatinized starch, dicalcium phosphate, and calcium carbonate; oxidation reaction enhancers such as elemental chromium, manganese, or copper, compounds comprising said elements, or mixtures thereof; hydrogen gas inhibitors such as inorganic or organic alkali compounds or alkali weak acid salts including sodium hydroxide, potassium hydroxide, sodium hydrogen carbonate, sodium carbonate, calcium hydroxide, calcium carbonate, and sodium propionate; fillers such as natural cellulosic fragments including wood dust, cotton linter, and cellulose, synthetic fibers in fragmentary form including polyester fibers, foamed synthetic resins such as foamed polystyrene and polyurethane, and inorganic compounds including silica powder, porous silica gel, sodium sulfate, barium sulfate, iron oxides, and alumina; and anti-caking agents such as tricalcium phosphate and sodium silicoaluminate. Such components also include thickeners such as cornstarch, potato starch, carboxymethylcellulose, and alpha-starch, and surfactants such as those included within the anionic, cationic, nonionic, zwitterionic, and amphoteric types. The preferred surfactant, if used however, is nonionic. Still other additional components which may be added to the particulate exothermic compositions of the present invention, as appropriate, include extending agents such as metasilicates, zirconium, and ceramics.

Preferably at least 50%, more preferably 70%, even more preferably 80% and most preferably 90% of all of the particles, by weight of the exothermic composition, have a mean particle size of less than 200 microns, preferably less than 150 microns.

The above-mentioned components of the composition are blended using conventional blending techniques. Suitable methods of blending these components are described in detail in U.S. Patent 4,649,895.

Alternatively to the above described particulate exothermic composition, the exothermic composition may be formed into agglomerated granules, direct compacted into compaction articles such as granules, pellets, tablets, and/or slugs, and mixtures thereof, which may be referenced as agglomerated pre-compaction compositions. As used herein, the term "agglomerated pre-compaction composition" means the mixture of dry powdered ingredients, comprising iron powder, carbonaceous powder, metal salt(s), water-holding agent(s), agglomeration aid(s), and dry binder(s) prior to direct compaction. As used herein, the term "direct compacted" or "direct compaction" means a dry powder mixture is or has been blended, compressed, and formed into pellets, tablets, or slugs without the use of typical wet binders/solutions to adhere the particulate(s) together. Alternatively, the dry powder mixture is, or has been, blended and roll compacted or slugged, followed by milling and screening, creating directly compacted granules. Direct compaction may also be known in the art as dry compaction. Other suitable methods of making tablets and/or slugs are described in detail in Chapter 89, "Oral Solid Dosage Forms," Remington's Pharmaceutical Sciences, 18th Edition, (1990), pp. 1634-1656.

The thermic cells can have any geometric shape, e.g., disk, triangle, square, cube, rectangle, cylinder, ellipsoid and the like, all or none of which may contain a hole through the middle or other reservoir.

The preferred shape of the cell comprises an ellipsoid geometry. In a preferred embodiment, the ellipsoid shapes may have a width at its widest point of from 0.15 cm to 20 cm, preferably from 0.3 cm to 10 cm, more preferably from 0.5 cm to 5 cm, most preferably from 1 cm to 3 cm, a height at its highest point of from 0.1 cm to 5 cm, preferably from 0.2 cm to 1 cm, more preferably from 0.2 cm to 0.8 cm, and most preferably from 0.2 cm to 0.7 and a length at its longest point of from 0.5 cm to 20 cm, preferably from cm to 15 cm, more preferably from cm to 10 cm, most preferably from 3 cm to 5 cm.

Alternatively, cells having geometric shapes other than an ellipsoid shape, preferably a disk shape may be used. The preferred disk shapes preferably have a cell diameter of from 0.2 cm to 10 cm, preferably from 0.5 cm to 8 cm, more preferably from cm to 5 cm, and most preferably from 1.5 cm to 3 cm. Cells preferably have a height of from 0.1 cm to 1 cm, preferably from greater than 0.1 cm to 0.9 cm, more preferably from greater than 0.2 cm to 0.8 cm, and most preferably from greater than 0.2 cm to 0.7 cm.

The compaction articles are preferably compressed to a mechanical strength which is capable of withstanding the shocks of handling in their manufacture, packing, shipping, and dispensing. The compaction articles are typically compressed to a density of greater than 1 $g/cm^3$, preferably from 1 $g/cm^3$ to 3 $g/cm^3$, more preferably from 1.5 $g/cm^3$ to 3 $g/cm^3$, and most preferably from 2 $g/cm^3$ to 3 $g/cm^3$.

In a preferred embodiment, the ratio of fill volume to cell volume of a given cell is from 0.7 to 1.0, preferably from 0.75 to 1.0, more preferably from 0.8 to 1.0, even more preferably from 0.85 to 1.0 and most preferably from 0.9 to 1.0. As used herein, the term "fill volume" means the volume of the particulate composition or the compacted, water-swelled, heating clement in the filled cell. As also used herein, the term "cell volume" means the fill volume plus the void volume of the cell. As also used herein, the term "void volume" means the volume of the cell left unfilled by the particulate composition or the compacted, water-swelled, heating element in a finished heat cell, not including the unfilled space within a tablet comprising a hole or reservoir, in a finished heat cell, measured without differential pressure in the cell and without additional stretching or deformation of the substrate material.

Oxygen permeability, allowing enhancement of the exothermic or endothermic reaction, may optionally be provided by selecting materials for the previously described base material and/or cover material that have the specifically desired permeability properties. The desired permeability properties may be provided by microporous films or by films which have pores or holes formed therein. The formation of these holes/pores may be via extrusion cast/vacuum formation or by hot needle aperturing. Oxygen permeability can also be provided in the present invention by perforating at least one of the base material and cover material with aeration holes using, for example, an array of pins having tapered points and diameters of from 0.2 mm to 2 mm, preferably from 0.4 mm to 0.9 mm. The array of pins is patterned such that the base material and/or cover material are perforated by from 10 to 30 pins per square centimeter. Alternatively, after the base material and cover material have been bonded together, enclosing the exothermic composition in the pocket between them, at least one side of the cell may be perforated with aeration holes using, for example, at least one pin, preferably an array of from 20 to 60 pins having tapered points and diameters of from 0.2 mm to 2 mm, preferably from 0.4 mm to 0.9 mm. The pins are pressed through one side of the base material and/or cover material to a depth of from 2% to 100%, preferably from 20% to 100%, and more preferably from 50% to 100% into the exothermic composition. This hole configuration provides an oxygen diffusion into the cell during oxidation of the endothermic or exothermic composition of from 0.01 ce $O_2$/min./5 $cm^2$ to 15.0 cc $O_2$/min./5 $cm^2$ (at 21°C, 1 ATM), preferably from 0.9 cc $O_2$/min./5 $cm^2$ to 3 cc $O_2$/min./5 $cm^2$ (at 21°C, 1 ATM).

The velocity, duration, and temperature of the thermogenic oxidation reaction of the endothermic or exothermic composition can be controlled as desired by changing the area of contact with air, more specifically, by changing the oxygen diffusion/permeability.

In a preferred embodiment, the thermic devices comprise at least one continuous layer of a material which exhibits the thermophysical characteristics specified herein. Continuous layer or layers of one or more such materials are typically included as one or both of the layers used to form the heat cells. Alternatively, the heat cells may be mounted individually or in one or more groups to one or more continuous layers of a material which exhibits the thermophysical characteristics specified herein.

The thermic devices of the present invention may optionally incorporate a component, such as a separate substrate layer or incorporated into at least one of the continuous layers, comprising active aromatic compounds, non-active aromatic compounds, pharmaceutical actives or other therapeutic agents, and mixtures thereof, to be delivered through the skin. Such active aromatic compounds include, but are not limited to, menthol, camphor, and eucalyptus. Such non-active aromatic compounds include, but are not limited to, benzaldehyde, citral, decanal, and aldehyde. Such pharmaceutical actives/therapeutic agents include, but are not limited to antibiotics, vitamins, antiviral agents, analgesics, anti-inflammatory agents, antipruritics, antipyretics, anesthetic agents, antifungals, antimicrobials, and mixtures thereof. The thermic device may also comprise a separate substrate layer, or incorporated into at least one of the continuous layers, a self-adhesive component and/or a sweat-absorbing component.

The thermic devices of the present invention may comprise any number of sizes and/or shapes, as appropriate, and may be used alone or can be incorporated into various wraps or pads. Typically, these wraps have a means for retaining wraps or pads in place around various parts of the body, such as knee, neck, back, abdomen, and the like, and can comprise any number of styles and shapes.

The finished thermic devices are typically packaged in a secondary package. An air-impermeable package may be used to prevent an oxidation reaction from occurring until desired as described in U.S. Patent No. 4,649,895. Alternatively, other means may also be used to prevent an oxidation reaction from occurring before desired, such as air impermeable removable adhesive strips can be placed over the aeration holes in the heat cells such that; when the strips are removed, air is allowed to enter the heat cell, thus activating the oxidation reaction of the iron powder.

Attachment Means

The present kits comprise means for releasably attaching the thermic device to one of the body compresses. As used herein, the term "releasably attaching" refers to that property which provides for initial permanent attachment of the thermic device to one of the body compresses and subsequent detachment of the thermic device from the body compress. The user may desire to release the thermic device and replace or reposition the thermic device for optimal thermic delivery to the desired area of the body. Accordingly, further releasable attachment of the thermic device to the body compress is often desirable, which may return the thermic device to the initial configuration relative to the body compress or may result in a repositioning of the attachment from the initial configuration. As used herein, the term "permanent attachment" refers to joining the thermic device to the body compress in such a manner that these two components remained physically joined during ordinary use.

Such means are integrated into the thermic device itself. The means comprise pressure sensitive adhesives.

In a preferred embodiment herein, the means for releasably attaching the thermic device to one of the body compresses includes narrow pressure sensitive adhesive strands or fibrils attached to the outer surface of the thermic device. For example, the thermic device may comprise release paper attached to adhesive strands on the outer layer of the thermic device in order to protect the adhesive strands from prematurely adhering to a target other than the intended body compress. Such means preferably has a stronger bond to the outer surface of the thermic device than to either the release paper or, upon use, to the target body compress surface. This may optionally be achieved by melting the adhesive into the material of the outer surface of the thermic device such that mechanical entanglement occurs.

In this embodiment, a preferred adhesive is grade #70-4595 pressure sensitive hot melt adhesive, commercially available from National Starch and Chemical Company of Bridgewater, NJ. As an example, this adhesive may be applied to the outer surface of the thermic device by slot die coating or printing. In either case it is desirable that the adhesive penetrate into the outer surface of the thermic device such that the adhesive preferentially adheres to the outer surface of the thermic device upon removal of the device from the body compress subsequent to use. The pattern of adhesive produced by this method may be straight parallel stripes extending from the edges of the thermic device. The release paper then utilized is preferably a a silione treated paper, such as 24 KSD release paper, commercially available from Tekkote of Leonia, NJ.

Example

The following provides a non-limiting example of the present invention. A kit comprising one body compress and four thermic devices is provided to a mammalian user. The thermic devices are devices in accordance with U.S. Patent No. 6,146,732. Each of the four thermic devices is separately packaged in a secondary package to inhibit initiation of the exothermic reaction until desired use. The body compress is the knee wrap of Fig. 1, previously described herein.

Referring further to Fig. 1, each of the thermic devices is substantially in the shape of first attachment zone 11 or second attachment zone 12 and is loaded with a pressure sensitive adhesive as described above with respect to the attachment means. Each of the thermic devices further comprises a release paper, in order to protect the adhesive prior to use. The thermic devices are removed from their respective secondary packaging and the release papers are removed. The first thermic device is releasably attached to the body compress by lightly pressing the outer surface of the thermic device (loaded with pressure sensitive adhesive) onto first attachment zone 11, while the second thermic device is similarly releasably attached to second attachment zone 12. Over a twenty minute period, the heat cells present in the thermic devices warm the device to a soothing temperature of about 41 °C. The mammalian user places the releasably attached body compress and thermic devices around the knee, with the heat cells proximal to the surface of the user's skin. The body compress is secured around the knee such that the user may perform ordinary daily activities.

After about twelve hours, the exothermic composition of the heat cells is substantially expended. The mammalian user removes the releasably attached body compress and thermic devices from the knee. The thermic devices are easily released from the body compress. The thermic devices are disposed of, while the body compress is either machine washed or associated again with the remaining thermic devices of the kit (*i.e.,* replaced inside the kit packaging), until further use.

## Claims

**1.** A kit comprising :

>   (a) one or more body compresses; and
>   (b) one or more thermic devices each comprising one or more exothermic or endothermic thermic cells;
>
>   **characterized in that** the thermic devices comprise a pressure sensitive adhesive for releasably attaching one or more of the thermic devices to one of the body compresses.

**2.** The kit according to Claim 1 wherein the thermic device is disposable.

**3.** The kit according to any of the preceding claims wherein the thermic device comprises a plurality of thermic cells.

**4.** The kit according to any of the preceding claims **characterized by** a plurality of thermic devices.

**5.** The kit according to any of the preceding claims wherein the thermic cells each, independently, comprise an exothermic composition wherein the exothermic composition comprises:

>   (a) from 30% to 80% of iron powder, by weight of the exothermic composition; and
>   (b) from 3% to 25% of carbonaceous material selected from the group consisting of activated carbon, non-activated carbon, and mixtures thereof, by weight of the exothermic composition.

**6.** The kit according to any of the preceding claims wherein one or more of the body compresses is selected from the group consisting of knee wraps, neck wraps, back wraps, menstrual pain relief compresses, and combinations thereof,

## Patentansprüche

**1.** Set, umfassend:

>   (a) eine oder mehrere Körperkompressen und
>   (b) eine oder mehrere thermische Vorrichtungen, die jeweils eine oder mehrere exotherme oder endotherme thermische Zellen umfassen;
>
>   **dadurch gekennzeichnet, dass** die thermischen Vorrichtungen einen Haftkleber zum lösbaren Befestigen einer oder mehrerer der thermischen Vorrichtungen an einer der Körperkompressen umfassen.

**2.** Set nach Anspruch 1, wobei die thermische Vorrichtung eine Einwegvorrichtung ist.

**3.** Set nach einem der vorstehenden Ansprüche, wobei die thermische Vorrichtung mehrere thermische Zellen umfasst.

**4.** Set nach einem der vorstehenden Ansprüche, das durch mehrere thermische Vorrichtungen gekennzeichnet ist.

**5.** Set nach einem der vorstehenden Ansprüche, wobei die thermischen Zellen jeweils, unabhängig voneinander, eine exotherme Zusammensetzung umfassen, wobei die exotherme Zusammensetzung Folgendes umfasst:

>   (a) von 30 Gew.-% bis 80 Gew.-% der exothermen Zusammensetzung Eisenpulver und
>   (b) von 3 Gew.-% bis 25 Gew.-% der exothermen Zusammensetzung kohleartiges Material, ausgewählt aus der Gruppe, bestehend aus Aktivkohle, nichtaktiviertem Kohlenstoff und Mischungen davon.

**6.** Set nach einem der vorstehenden Ansprüche, wobei eine oder mehrere der Körperkompressen ausgewählt sind aus der Gruppe, bestehend aus Kniewickeln, Halswickeln, Rückenwickeln, Menstruationsschmerz lindernden Kompressen und Kombinationen davon.

## Revendications

**1.** Trousse comprenant :

>   (a) une ou plusieurs compresses corporelles ; et
>   (b) un ou plusieurs dispositifs thermiques, chacun comprenant une ou plusieurs alvéoles thermiques exothermiques ou endothermiques ;
>
>   **caractérisée en ce que** les dispositifs thermiques comprennent un adhésif sensible à la pression pour fixer de façon libérable un ou plusieurs dispositifs thermiques à une des compresses corporelles.

**2.** Trousse selon la revendication 1, dans laquelle le dispositif thermique est jetable.

**3.** Trousse selon l'une quelconque des revendications précédentes, dans laquelle le dispositif thermique comprend une pluralité d'alvéoles thermiques.

**4.** Trousse selon l'une quelconque des revendications précédentes, **caractérisée par** une pluralité de dispositifs thermiques.

**5.** Trousse selon l'une quelconque des revendications précédentes, dans laquelle les alvéoles <u>thermiques</u> comprennent chacune, indépendamment, une composition exothermique dans laquelle la composition exothermique comprend :

> (a) de 30 % à 80 % de poudre de fer, en poids de la composition exothermique ; et
> (b) de 3 % à 25 % de matériau carboné choisi dans le groupe constitué de charbon actif, charbon non-actif, et leurs mélanges, en poids de la composition exothermique.

**6.** Trousse selon l'une quelconque des revendications précédentes, dans laquelle une ou plusieurs des compresses corporelles est choisie dans le groupe constitué de genouillères, collerettes, corsets, compresses de soulagement des douleurs menstruelles, et leurs combinaisons.

Fig. 1